# FASCICULE DE BREVET EUROPEEN

(11) **EP 2 113 286 B1**
(45) Date de publication et mention de la délivrance du brevet: **30.01.2013**
(21) Numéro de dépôt: 09159080.2
(22) Date de dépôt: 29.04.2009
(51) Int. Cl.: A61Q 5/00, A61K 8/64, A61K 8/60, C07K 14/47

(54) **Utilisations de BNIPXL-beta dans la canitie précoce**
Verwendungen von BNIPXL-beta in der Behandlung von frühzeitiger Haarergrauung
Uses of the BNIPXL-beta in early canities

(30) Priorité: 30.04.2008 FR 0802435
(43) Date de publication de la demande: 04.11.2009
(73) Titulaire: L'Oréal, 75008 Paris (FR)
(72) Inventeur: Bernard, Bruno, 92400 Courbevoie (FR); Commo, Stéphane, 92370 Chaville (FR); De Lacharriere, Olivier, 75015 Paris (FR)
(74) Mandataire: Sellin, Carole

(56) Documents cités:
- WO-A-2004/007742
- QIN W ET AL: "BNIPL-2, a novel homologue of BNIP-2, interacts with Bcl-2 and Cdc42GAP in apoptosis" BIOCHEMICAL AND BIOPHYSICAL RESEARCH COMMUNICATIONS, ACADEMIC PRESS INC. ORLANDO, FL, US, vol. 308, no. 2, 22 août 2003 (2003-08-22), pages 379-385, XP004442987 ISSN: 0006-291X
- ZHOU Y T ET AL: "BNIP-2 induces cell elongation and membrane protrusions by interacting with Cdc42 via a unique Cdc42-binding motif within its BNIP-2 and Cdc42GAP homology domain" EXPERIMENTAL CELL RESEARCH, ACADEMIC PRESS, US, vol. 303, no. 2, 15 février 2005 (2005-02-15), pages 263-274, XP004709266 ISSN: 0014-4827
- MACHIDA T ET AL: "Increased expression of proapoptotic BMCC1, a novel gene with the BNIP2 and Cdc42GAP homology (BCH) domain, is associated with favorable prognosis in human neuroblastomas" ONCOGENE, vol. 25, no. 13, mars 2006 (2006-03), pages 1931-1942, XP002502656 ISSN: 0950-9232

## Description

Annuler ou atténuer autant que faire se peut les effets du vieillissement est une préoccupation qui ne cesse de croître. Dans ce contexte, faire disparaître des cheveux blancs jugés disgracieux grâce à des shampoings traitants colorants est désormais une activité très répandue. Il est clair cependant que, même si cette technique permet effectivement d'annuler les effets du phénomène, elle n'en affecte nullement les causes. De ce fait, cette solution est temporaire et doit être fréquemment renouvelée.

Le blanchissement du cheveu (canitie) est dû à une disparition progressive des mélanocytes du follicule pileux (Commo S., Gaillard, O. & Bernard, B. A., 2004). Ce processus affecte à la fois les mélanocytes de l'unité de pigmentation situés à la base du follicule et responsables directs de la pigmentation de la fibre, ainsi que les mélanocytes progéniteurs situés dans la partie distale de la gaine externe du follicule pileux et qui servent de réservoir à partir duquel l'unité de pigmentation est renouvelée à chaque cycle pilaire (Commo, S. & Bernard, B.A. 2000).

Bien que cette disparition progressive des mélanocytes semble liée à l'absence d'expression de l'enzyme dopachrome tautomérase (Commo S., Gaillard O., Thibaut S. & Bernard B.A, 2004, brevets FR2840530, FR2840531, FR2863484), les inventeurs ont choisi d'explorer l'apparition des cheveux blancs, ou **canitie**, sous un angle totalement nouveau, celui de la génétique. Ils ont cherché à identifier par une approche génétique globale d'autres gènes éventuellement associés à la canitie précoce.

En effet, explorer la canitie de par son aspect génétique permet de mettre en évidence les mécanismes profonds de la dépigmentation. Cela permet aussi d'identifier les gènes qui sont impliqués dans la canitie. Cette identification ouvre la porte à de très nombreuses applications, dans le domaine du soin des cheveux, tant cosmétiques que thérapeutiques ou diagnostiques.

A cette fin, les inventeurs ont choisi de focaliser leurs recherches dans un premier temps sur la canitie précoce (CP), ou apparition de cheveux blancs très tôt dans la vie, dont le caractère héréditaire est connu et qui est lié à certaines maladies auto-immunes. Pour explorer la canitie de par son aspect génétique, il a été réalisé une étude de ségrégation de l'ADN chez des familles dont la canitie apparaît très tôt dans la vie. Afin de garantir les meilleures chances de succès pour cette chasse aux gènes, le phénotype CP fut attribué aux seuls individus qui présentaient des cheveux blancs avant 25 ans et dont la moitié de la chevelure était grise à 30 ans. Douze familles ont été retenues pour participer à une étude de liaison.

Les résultats de ces travaux ont permis aux inventeurs de définir dans un premier temps des zones chromosomiques et/ou génomiques comprenant des gènes impliqués avec une forte probabilité dans la canitie. Cinq loci ont ainsi été identifiés sur les chromosomes 3, 5 et 11 (demandes de brevet FR2842105 et WO04/007764) et sur les chromosomes 6 et 9 (demandes de brevet FR2842104, FR2853532, FR2865217 et WO04/007742).

Parmi les gènes associés à la canitie précoce, le gène DDX31 (GI : 20336296 ; accession n° NM_022779) a été proposé comme gène putatif lié à la canitie précoce, situé en 9q34 et décrit dans les demandes de brevets L'Oréal FR2853532 et FR2865217. DDX31 est une probable hélicase à ARN ATP-dépendante, appartenant à la famille des hélicases à motif DEAD (« DEAD-box protein 31 »).

Afin d'explorer plus avant le rôle de DDX31 dans la processus de canitie précoce, les inventeurs ont cherché à identifier, par une approche double hybride dans le système levure, les éventuels partenaires protéiques susceptibles d'interagir avec DDX31.

De façon surprenante et inattendue, les inventeurs ont découvert que BNIPXL-beta (BCI2/adenovirus E1B 19kDa protein-interacting protein 2) était une proie de DDX31, préférentiellement de la forme longue de DDX31. Le score de confiance dans l'interaction est particulièrement bon.

Les différentes fonctions de BNIPXL-beta sont encore mal connues. Cette protéine est produite par épissage alternatif de KIAA0367 (alias: BCH motif-containing molecule at the C-terminal region 1, BNIP2 motif-containing molecule at the C-terminal region 1; n°accession Q58A63 ; GI : 125987727).

Par homologie de domaines (domaine CRAL-TRIO ou Sec14 ; domaine présent dans BNIP2), il est supposé que BNIPXL-beta est impliqué dans le transport et le métabolisme de phospholipides ;qu'il a une fonction pro-apoptotique lié à une interaction avec bcl2 ; et qu'il participe au trafic cellulaire ; il pourrait jouer un rôle dans la formation de protrusions membranaires (Machida *et al,* 2006; Zhou *et al,* 2005; Shang *et al,* 2003; Qin *et al,* 2003; Belcredito *et al,* 2001; Sirokmany *et al*, 2006; Mousley *et al,* 2007).

L'interaction entre DDX31 et BNIPXL-beta est donc très vraisemblablement importante dans le trafic vésiculaire, d'où son rôle dans la migration et le transfert des mélanosomes et par là dans l'implication dans la canitie dont la canitie précoce.

Dans cette demande, les termes qui suivent ont plus particulièrement la signification suivante :
**Degré d'identité** : le degré d'identité entre deux séquences (protéiques ou nucléiques) est déterminé notamment en alignant les deux séquences afin de maximiser les points de concordance tout en minimisant les espaces (« gap ») ; il est obtenu en divisant le nombre d'acides aminés ou d'acides nucléiques communs par la longueur de la plus longue des deux séquences.
**ARNi** (RNAi en anglais) : ARN interférence : une molécule d'ARN capable de cibler une séquence particulière dans une molécule d'ARN et de guider le clivage de cet ARN à un site déterminé au sein de la séquence cible. La molécule d'ARN capable de cliver par ARN interférence est, par extension, dénommée ARN interférent ou ARNi. Quand cette réaction a lieu à l'intérieur d'une cellule et que l'ARN clivé est de l'ARN messager (ARNm), le clivage de la molécule d'ARNm conduit ensuite à la dégradation de la molécule, empêchant ainsi toute étape ultérieure, telle que la traduction de l'ARNm en protéine. Selon le type d'ARNi, la séquence cible est soit la séquence complémentaire de l'ARN interférent (notamment siRNA) soit une séquence quasi-complémentaire de l'ARN interférent, avec toutefois quelques positions non concordantes (notamment miRNA).
**siRNA:** short interfering RNA, anglicisme signifiant ARN interférent de petite taille. Il s'agit d'une séquence d'ARN double brin, comprenant environ de 21 à 23 nucléotides (Dykxhoorn et al, Nature Reviews 2003, vol 4, p.457). Les siRNA peuvent provenir du clivage d'ARN double brin de taille plus importante par une protéine dénommée dicer. Les siRNA ainsi produits sont ensuite intégrés dans un complexe multiprotéique d'inhibition induite par l'ARN (RISC RNA-inducing silencing complex). L'ARNm cible est clivé en un site unique au milieu de la région duplex formée entre le siRNA (qui sert de guide) et l'ARNm cible, à 10 nucléotides de l'extrémité 5' du siRNA.

Afin d'obtenir des siRNA, dans une cellule, plusieurs méthodes sont envisageables.
1. il est possible d'introduire directement dans la cellule des siRNA obtenus par synthèse chimique ; dans ce cas, l'action du dicer est contournée.
2. il est également envisageable d'introduire dans la cellule des fragments d'ARN double-brin de grande taille, qui sont ensuite clivés en siRNA par la protéine dicer.
3. il est possible d'introduire ou d'exprimer dans la cellule un duplex d'ARN en épingle à cheveux, par exemple un shRNA (pour small ou short haipin RNA), qui est ensuite clivé par la protéine Dicer en siRNA.

Par **fragment polynucléotidique,** on entend toute molécule résultant de l'enchaînement linéaire d'au moins deux nucléotides, cette molécule pouvant être monocaténaire, bicaténaire ou tricaténaire. Il peut donc s'agir d'une molécule d'ADN double-brin, d'un ADN simple brin, d'un ARN, d'un duplex ADN simple brin-ARN, d'un triplex ADN-ARN ou de toute autre combinaison. Le fragment polynucléotidique peut être naturel isolé, recombinant ou bien synthétique. Lorsque le fragment polynucléotidique comporte des brins complémentaires, la complémentarité n'est pas nécessairement parfaite, mais l'affinité entre les différents brins est suffisante pour permettre l'établissement de liaison stable de type Watson Crick entre les deux brins.

Bien que l'appariement des bases soient de préférence de type Watson-Crick, d'autres types ne sont pas exclus, tels qu'un appariement de type Hoogsteen ou Hoogsteen inverse. Selon un premier aspect, la présente invention concerne la protéine BNIPXLβ, ou une partie de BNIPXLβ, pour une application thérapeutique ou cosmétique, notamment pour le traitement ou la prévention de la canitie précoce chez l'homme. En effet, du fait de son interaction avec DDX31, dans un processus impliqué dans la canitie précoce, cette protéine est elle-même en rapport avec la canitie, notamment la canitie précoce.

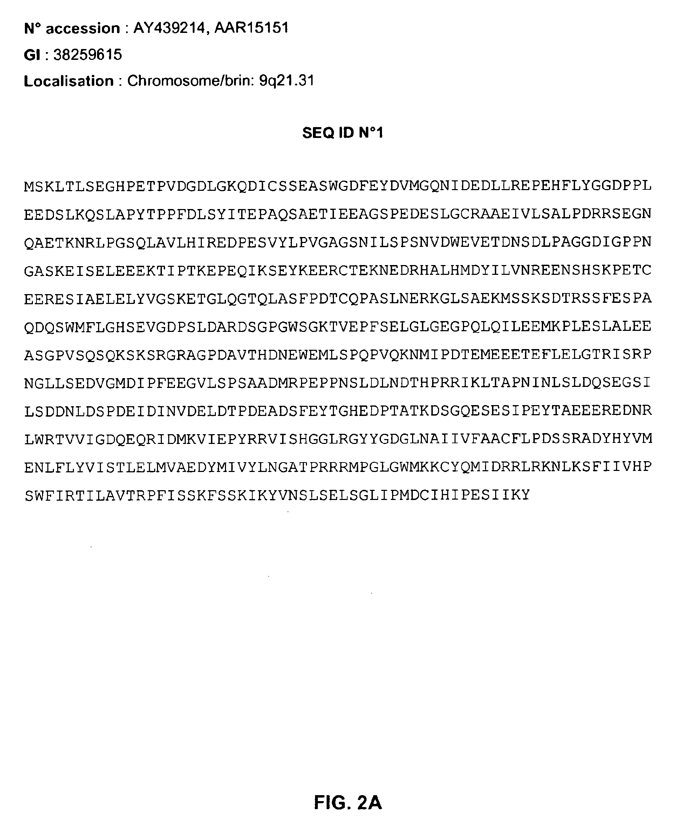

La séquence de la protéine BNIPXLβ est illustrée à la figure 2A et correspond à la séquence SEQ ID N°1. Une partie de BNIPXLβ, dans le cadre de la présente demande, est un polypeptide comprenant au moins 30 acides aminés consécutifs de SEQ ID N°1.

Dans tout le cadre de cette description, on entend par cosmétique toute application qui ne tend à modifier que l'esthétique et n'a aucune visée thérapeutique.

L'invention concerne également un polypeptide comprenant la séquence de BNIPXLβ ou comprenant une partie de BNIPXLβ, ladite partie étant telle que définie précédemment, pour une application thérapeutique ou cosmétique.

L'invention concerne également tout polypeptide comprenant une région ayant au moins 90% d'identité avec BNIPXLβ, ou avec une partie de BNIPXLβ, pour une application thérapeutique, notamment dans le domaine de la canitie précoce. Ladite partie de BNIPXLβ comprend au moins 30 acides aminés.

Le polypeptide décrit comprend donc une région dont la séquence partage un fort pourcentage d'identité avec BNIPXLβ ou avec une partie de BNIPXLβ, mais il peut également comprendre d'autres séquences, en sus de celle mentionnée précédemment, à l'extrémité N-terminale ou bien C-terminale, ou bien aux deux.

De préférence, dans la région du polypeptide présentant un fort pourcentage d'identité avec BNIPXLβ ou avec une partie de BNIPXLβ, le pourcentage d'identité est supérieur à 90%, de préférence même au-delà de 95%, voire 98%.

Selon une mise en oeuvre préférée, ledit polypeptide comprend une partie de la protéine BNIPXLβ, cette partie comprenant au moins 30 acides aminés ou plus.

De manière préférée également, le polypeptide comprend une séquence partageant un fort pourcentage d'identité avec une partie de BNIPXLβ d'au moins 40, voire 50 acides aminés consécutifs, voire même 75 ou 100, sur les 732 acides aminés de la protéine BNIPXLβ. De préférence, la partie de BNIPXLβ est une partie correspondant à un domaine ayant une importance biologique, par exemple un site de fixation avec un partenaire protéique. Il existe des programmes bien connus de l'homme du métier pour déterminer des domaines particulièrement intéressants au sein de la séquence SEQ ID N°1.

Les applications thérapeutiques ou cosmétiques mentionnées sont dans la thérapie du cheveu, notamment le traitement ou la prévention de la canitie précoce, de préférence héréditaire.

La présente invention concerne également l'utilisation de BNIPXLβ, d'une partie de BNIPXLβ telle que décrite plus haut ou d'un polypeptide tel que décrit, pour la préparation d'un médicament pour le traitement ou la prévention de la canitie précoce chez l'homme.

Par ailleurs, les inventeurs ont mis en évidence l'importance de l'interaction entre DDX31 et BNIPXLβ, et par là l'effet bénéfique d'une diminution du niveau d'expression de BNIPXLβ, qui peut être obtenue par ARNi. Un tel effet bénéfique peut être notamment dans le phénotype canitie précoce.

La présente invention concerne donc également une molécule comprenant une séquence d'ARNi ayant au moins 90% d'identité avec tout ou partie de la séquence d'ARNm correspondant à BNIPXLβ, pour un usage thérapeutique ou cosmétique chez l'homme, ladite partie comprenant au moins 18 nucléotides. De préférence, la partie en question comprend au moins 20, 25 voire 30 nucléotides.

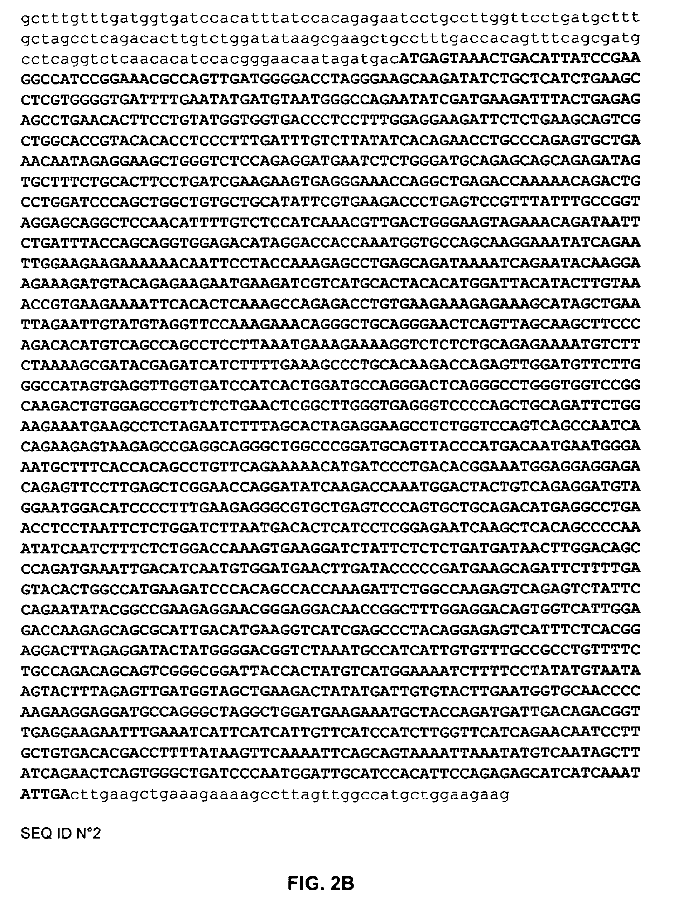

L'application thérapeutique consiste au traitement ou à la prévention de la canitie précoce. La séquence d'ARNm est équivalente à la séquence d'ADNc de BNIPXLβ, illustrée à la figure 2B, en tant que séquence SEQ ID N°2.

Dans le cadre de la présente demande, lors de la détermination du degré d'identité entre une séquence d'ARN et une séquence d'ADN, il est considéré que la guanine de l'ARN est équivalente à la thymine de l'ADN ; par conséquent, une séquence d'ADN donnée d'un part, et la séquence d'ARN issue de cette séquence d'ADN en remplaçant les T par des G d'autre part, présentent 100% d'identité au sens de la présente demande.

L'ARNi sus-mentionné consiste de préférence en un fragment d'ARN double-brin, les deux séquences appariées pouvant se trouver au sein d'une même molécule, formant ainsi une structure en épingle à cheveux, ou bien au sein de deux molécules d'ARN simple brin distinctes.

La molécule comprenant la séquence d'ARNi, peut donc être constituée d'un seul fragment d'ARN se repliant ou bien de deux molécules d'ARN. Cette molécule peut comprendre d'autres séquences, d'ARN ou d'ADN, simple ou double brin, du côté 3', 5' ou bien des deux côtés des séquences s'appariant, ou également entre les séquences s'appariant, par exemple dans la boucle pour le cas où la molécule est constituée d'une seule molécule d'ARN s'appariant.

L'ARNi mentionné peut être un siRNA ou un shRNA. De préférence, la molécule comprenant la séquence d'ARNi est telle qu'elle peut être clivée par la protéine Dicer afin de donner un shRNA interférant avec l'ARNm de BNIPXLβ.

La présente invention concerne aussi l'utilisation d'une molécule telle que définie précédemment, pour la préparation d'un médicament pour le traitement ou la prévention de la canitie précoce chez l'homme.

L'invention concerne de plus des procédés de criblage et notamment un procédé de criblage de molécules modulant l'expression du gène codant pour BNIPXL-beta afin d'identifier un agent pour une utilisation à des fins cosmétiques ou thérapeutiques, dans le domaine de la pigmentation.

Un gène codant pour la protéine BNIPXLβ (ou BNIPXL-beta) est par exemple le gène BMMCC1 (synonymes BNIPXL, KIAA0367) dont un des numéros d'accession dans les bases est ENSG00000106772 (Ensemble Gene ID). De ce gène, par épissage alternatif, résulte au moins trois variants ; l'isoforme 1 canonique correspond à un polypeptide de 2724 acides aminés ; l'isoforme 3 correspond à BNIPXL-béta, polypeptide de 732 acides aminés se distinguant essentiellement de l'isoforme 1 canonique par l'absence des acides aminés 1-1959.

La modulation de l'expression d'un gène peut provenir notamment de la modulation de sa transcription ou de sa traduction.

Un criblage selon le procédé de l'invention permet donc de mettre en évidence des molécules qui empêchent, inhibent ou retardent la transcription et/ou la traduction du gène, ou bien qui augment ou diminuent le niveau de transcription et/ou de traduction du gène, ou bien qui modifient les proportions des différents isoformes dans l'expression du gène BMMCC1. Une molécule susceptible d'être identifiée par le crible selon l'invention est donc par exemple une molécule diminuant considérablement l'expression de l'isoforme 3 au profit de l'isoforme 1 du gène BMMCC1.

Par augmentations, diminutions, modulations, inhibitions ou modulations, il faut comprendre des variations d'amplitude significative, c'est-à-dire supérieure au bruit et à l'imprécision de la mesure, donc des variations significatives d'un point de vue statistique, supérieures à la déviation standard.

Il est à noter qu'un procédé de criblage selon l'invention est spécialement conçu pour être mis en oeuvre *ex vivo*, par exemple dans des cultures cellulaires. De manière préférentielle, il est mis en oeuvre *in vitro.*

Un procédé selon l'invention comprend de préférence les étapes suivantes :
- mise en présence de la molécule à tester et du gène codant pour BNIPXL-beta, dans des conditions permettant l'expression dudit gène, en l'absence de la molécule à tester et
- détection d'une variation dans le niveau d'expression dudit gène ou bien dans la nature des produits d'expression dudit gène, du fait de la présence de la molécule à tester.

On entend par 'nature des produits d'expression', comme explicité plus haut, par exemple les proportions respectives des différents isoformes, ou bien par exemple l'apparition ou la disparition d'un isoforme donné.

Les molécules à tester peuvent être de toute nature chimique. Il s'agit de préférence de molécules de petite taille, susceptible de traverser les parois cellulaires. Des molécules préférées sont notamment des molécules chimiques artificielles comprenant essentiellement les éléments C, H, O, N, avec éventuellement des éléments Cl ou F. Alternativement, les molécules à tester peuvent être des molécules biologiques, telles que des protéines, des polypeptides, des anticorps, des fragments d'anticorps, des ARNi, des ARN antisens, des ribozymes, des aptamères, molécules qui sont susceptibles d'être synthétisées à l'intérieur d'une cellule eucaryote.

Des molécules à tester particulièrement préférées sont des protéines, des ARNi, des ribozymes ou des ARN antisens ciblant BNIPXLβ ou ciblant le gène codant pour BNIPXL-beta, par exemple des anticorps dirigés contre BNIPXLβ, ou bien des ARNi contre l'ARNm correspondant à BNIPXLβ.

Les conditions permettant l'expression dudit gène impliquent notamment la présence des différentes enzymes nécessaires telles que l'ARN polymérase, des nucléotides et acides aminés, ribosomes, ou toute autre structure permettant la transcription et la traduction dudit gène. Ces différents éléments se trouvent naturellement au sein des cellules en règle générale et doivent être ajoutés si le procédé est mis en oeuvre *in vitro.*

La détection d'une variation dans le niveau d'expression dudit gène ou bien dans la nature des produits d'expression dudit gène, suite à l'addition de la molécule à tester, peut être réalisée par tout moyen considéré comme approprié par l'homme du métier. Il peut s'agir de dosage, par exemple dosage enzymatique, ou bien par utilisation de moyens de détection basés sur l'immunologie (dosage immunologique), ou bien par tout autre moyen connu basé par exemple sur les propriétés fonctionnelles des produits d'expression, leurs propriétés physico-chimiques (point isolélectrique, masse moléculaire, etc...), immunologiques, ou autres.

Les proportions des différents isoformes peuvent être déterminées sur le même principe. Il peut notamment être fait usage de différents anticorps reconnaissant spécifiquement un isoforme donné et n'interagissant pas avec les autres isoformes.

Dans le cadre de la présente invention, il doit être noté que la modulation de l'expression particulièrement recherchée est une inhibition de l'expression, notamment une inhibition de la transcription ou de la traduction du gène BMMCC1, ou bien une diminution de la proportion de l'isoforme 3 parmi les produits d'expression du gène BMMCC1.

L'inhibition peut être totale ou bien partielle ; dans tous les cas, elle implique une diminution de la production du polypeptide BNIPXLβ à partir du gène BMMCC1, ladite diminution étant significative d'un point de vue statistique. Il s'agit de préférence d'une diminution du niveau d'expression de BNIPXLβ à partir du gène BMMCC1 d'au moins 20%, de préférence d'au moins 35, voire 50%, et de préférence encore d'une diminution supérieure à 60%.

Un procédé tel que décrit précédemment peut bien entendu comprendre une ou plusieurs étapes additionnelles, antérieures, postérieures et/ou intermédiaires. Il est notamment envisagé, dans le cas où le procédé a permis de mettre en évidence une molécule modulant significativement l'expression du gène BMMCC1 de procéder à une étape d'identification de la molécule, puis éventuellement à la production en quantité plus importante de ladite molécule. Il peut également être envisagé de tester différentes autres molécules de structure très proche de celle permettant d'obtenir une modulation de l'expression dudit gène.

La présente invention concerne également un procédé de criblage de molécules capables de moduler l'activité du polypeptide BNIPXL-beta, pour l'identification d'un agent pour une utilisation à des fins cosmétiques ou thérapeutiques, dans le domaine de la pigmentation. Les molécules à tester peuvent être de toute nature comme explicité précédemment, ou bien parfaitement artificielles ou bien présentes naturellement dans des systèmes biologiques.

De même que le précédent procédé de criblage décrit, ce procédé de criblage est de préférence mis en oeuvre *ex vivo*, et de préférence *in vitro* .

Par modulation de l'activité, on entend toute modification significative des paramètres définissant l'activité, notamment la nature de la cible et la vitesse de réaction dans le cas d'une activité enzymatique et l'affinité et l'avidité dans le cas d'une interaction.

Le caractère « significatif » d'une modification a déjà été explicité précédemment, quand le paramètre qui est modifié est quantifiable.

Un procédé de criblage tel que décrit comprend de préférence les étapes suivantes :
- mise en présence de la molécule à tester et du polypeptide BNIPXL-beta, dans des conditions permettant la mise en évidence de l'activité dudit polypeptide en l'absence de la molécule à tester et
- détection d'une variation dans l'activité dudit polypeptide, du fait de la présence de la molécule à tester.

Les conditions permettant la mise en évidence de l'activité de BNIPXLβ impliquent notamment la présence des différents partenaires interagissant avec BNIPXLβ dans le cadre de cette activité, ainsi que les conditions physico-chimiques adaptées, telles que température, pH, caractère oxydant ou réducteur du milieu.

Ces différents éléments et conditions se trouvent naturellement au sein des cellules en règle générale, ou tout du moins de certaines, et doivent être ajoutés ou adaptés si le procédé est mis en oeuvre *in vitro.*

L'activité dont on détecte une variation peut être par exemple l'interaction avec DDX31, de préférence avec la forme longue.

Le polypeptide BNIPXL-beta utilisé dans le cadre de ce procédé est un polypeptide ayant la séquence SEQ ID N°1 et ayant de préférence subi les mêmes modifications post-traductionnelles que la protéine BNIPXLβ quand elle est produite in vivo dans des cellules humaines, notamment ayant le même profil de glycosylation. Cependant, dans le cadre du procédé de l'invention, en sus des polypeptides consistant en la séquence SEQ ID N°1, il peut aussi être envisagé d'utiliser des polypeptides ayant une séquence pas entièrement identique à SEQ ID N°1, soit parce qu'elle est plus longue mais qu'elle contient SEQ ID N°1, soit parce qu'elle est plus courte mais qu'elle comprend au moins 100 acides aminés consécutifs de SEQ ID N°1, soit parce qu'elle comprend une séquence ayant au moins 90% d'identité avec SEQ ID N°1 ou une partie de SEQ ID N°1, de préférence au moins 95% d'identité, ladite partie ayant de préférence au moins 50 acides aminés, voire au moins 100 acides aminés ; les différences par rapport à SEQ ID N°1 pouvent être cumulatives. Il est également envisagé d'utiliser un polypeptide dont les modifications post-traductionnelles ne soient pas identiques à celles de la protéine BNIPXLβ produite dans une cellule humaine. Dans le cas où le procédé de criblage de l'invention est mis en oeuvre avec une protéine présentant quelques modifications par rapport à la protéine BNIPXLβ humaine naturelle, l'homme du métier saura qu'il est important dans ce cas de confirmer la modulation observée par ce procédé en réitérant le procédé avec la protéine BNIPXLβ humaine naturelle.

La détection d'une variation dans l'activité dudit polypeptide, suite à l'addition de la molécule à tester, peut être réalisée par tout moyen considéré comme approprié par l'homme du métier. On peut détecter un paramètre lié directement à l'activité dudit polypeptide (par exemple apparition d'un produit résultant directement de l'activité enzymatique de BNIPXLβ) ou bien un paramètre lié indirectement à cette activité, notamment dans le cas de déclenchement de cascades enzymatiques.

Il peut s'agir de dosage, par exemple dosage enzymatique du produit de la réaction, ou par utilisation de moyens de détection basés sur l'immunologie (dosage immunologique) d'un complexe formé entre le polypeptide et sa cible, sa cible pouvant être DDX31, ou bien par tout autre moyen connu basé par exemple sur les propriétés fonctionnelles du produit résultant de l'activité du polypeptide, c'est-à-dire par exemple sur les propriétés physico-chimiques (point isolélectrique, masse moléculaire, etc...), immunologiques, ou autres d'un polypeptide ou complexe résultant de l'activité de BNIPXLβ.

Selon une mise en oeuvre préférée de ce procédé de l'invention, la modulation de l'activité de BNIPXLβ qui est recherchée est une inhibition, totale ou partielle, de cette activité.

Une inhibition partielle est de préférence une inhibition d'au moins 10% de l'activité dudit polypeptide par référence au niveau d'activité avant l'ajout de la molécule à tester.

De façon préférentielle, la modulation de l'activité est réalisée par séquestration dudit polypeptide. De cette manière, le polypeptide BNIPXLβ n'est plus disponible pour une autre interaction que celle avec la molécule séquestrante, ou bien est séquestré dans un compartiment cellulaire où ses partenaires d'interaction sont absents. L'homme du métier est en mesure de définir, sur la base de la structure et de la séquence de BNIPXLβ, les molécules ou les conditions susceptibles d'avoir l'effet de séquestration recherché.

Pour la mise en oeuvre du procédé de l'invention tel que décrit, il est particulièrement avantageux d'utiliser comme molécules à tester des anticorps dirigés contre le polypeptide BNIPXL-beta ou contre une partie de celui-ci. Des programmes permettent de définir quelle portion de BNIPXL-beta est susceptible d'être immunogène et donc de permettre l'obtention d'anticorps. Par ailleurs, la technologie des anticorps, monoclonaux et polyclonaux, est bien développée à l'heure actuelle, également concernant l'humanisation d'anticorps murins, ce qui permet sans peine à l'homme du métier de produire et de tester rapidement un grand nombre de molécules de cette catégorie dans un procédé selon l'invention.

Comme déjà mentionné précédemment, les procédés de criblage tels que décrits dans cette invention peuvent avantageusement comprendre une ou plusieurs étapes additionnelles en sus de celles déjà mentionnées, notamment des étapes antérieures, postérieures et/ou intermédiaires.

Pour les deux types de procédés de criblage qui ont été décrits, le but est l'identification, parmi les molécules testées, d'agent pour une utilisation à des fins cosmétiques ou thérapeutiques, dans le domaine de la pigmentation. La pigmentation dont il est question est la pigmentation des cheveux.

La présente invention concerne également diverses utilisations, notamment l'utilisation d'agents qui ont été criblés par les procédés précédemment décrits, à des fins cosmétiques, dans le domaine de la pigmentation, ou bien à des fins thérapeutiques, de traitement ou de prévention, dans le domaine de la pigmentation.

La présente invention a aussi trait à l'utilisation d'agent modulant l'expression du gène codant pour BNIPXL-beta, à des fins cosmétiques, dans le domaine de la pigmentation, ainsi qu'à l'utilisation d'un agent modulant l'expression du gène codant pour BNIPXL-beta, pour la fabrication d'un médicament pour une utilisation thérapeutique dans le domaine de la pigmentation.

La définition de ce qu'il faut entendre par les termes « moduler l'expression » a déjà été donnée, dans le cadre des procédés de criblage de l'invention. Ce terme couvre notamment la modulation de la transcription et la modulation de la traduction du gène en question. La modulation est de préférence une diminution, voire une inhibition, totale ou partielle, de ladite expression, transcription ou traduction, du gène.

Un agent correspond à tout type de molécules chimiques, naturelles ou artificielles. Des agents particulièrement préférés sont ceux qui sont susceptibles d'être identifiés ou qui ont été identifiés par un procédé de criblage selon l'invention tel que décrit précédemment, i.e. un procédé de criblage de molécule modulant l'expression du gène codant pour BNIPXLβ. La présente demande concerne de plus un agent modulant l'expression du gène codant pour BNIPXL-beta, pour une utilisation thérapeutique dans le domaine de la pigmentation. Sont également décrites les utilisations d'un agent modulant l'activité d'un polypeptide issu de la traduction du gène codant pour BNIPXL-beta, à des fins cosmétiques, dans le domaine de la pigmentation, ainsi que l'utilisation d'un agent modulant l'activité d'un polypeptide issu de la traduction du gène codant pour BNIPXL-beta, pour la fabrication d'un médicament pour une utilisation thérapeutique dans le domaine de la pigmentation.

De préférence, un polypeptide issu de la traduction du gène codant pour BNIPXL-beta est justement BNIPXL-beta.

La définition de ce qu'il faut entendre par les termes « moduler de l'activité » a déjà été donnée, dans le cadre des procédés de criblage de l'invention. La modulation est de préférence une diminution, voire une inhibition, totale ou partielle, de ladite activité.

Un agent modulant l'activité d'un polypeptide issu de la traduction du gène codant pour BNIPXL-beta, peut être tout type de molécule chimique, naturelle ou artificielle. Des agents particulièrement préférés sont ceux qui sont susceptibles d'être identifiés ou qui ont été identifiés par un procédé de criblage selon l'invention tel que décrit précédemment, i.e. un procédé de criblage de molécules capables de moduler l'activité du polypeptide BNIPXL-beta.

La présente demande divulgation décrit de plus un agent modulant l'activité de BNIPXL-beta, pour une utilisation thérapeutique dans le domaine de la pigmentation.

Selon un autre aspect, l'invention est aussi dirigée vers l'utilisation d'au moins un fragment polynucléotidique comprenant au moins 18 nucléotides consécutifs dont la séquence correspond à tout ou partie du gène codant pour BNIPXL-beta ou du transcrit BNIPXL-beta, à des fins cosmétiques, dans le domaine de la pigmentation.

Le fragment polynucléotidique dont il est fait référence dans le cadre de l'invention correspond à un fragment d'un chromosome. Ce fragment a une longueur minimale de 18 nucléotides, et une longueur maximale qui peut aller jusqu'à la longueur totale du gène codant pour BNIPXLβ. De préférence, le fragment a un nombre de nucléotides supérieur à 18. Une longueur particulièrement préférée est comprise entre 18 et 10000 nucléotides, de préférence entre 30 et 8000 nucléotides.

Selon des variantes préférées de l'invention, il est fait référence à des fragments dont la longueur est comprise entre 30 et 5000 nucléotides, de préférence entre 50 et 3000 nucléotides, par exemple entre 100 et 2000 nucléotides, ou entre 200 et 1000 nucléotides. Les présents inventeurs ont mis en évidence l'interaction entre le polypeptide BNIPXLβ et un polypeptide issu de la traduction du gène DDX31, cette interaction étant impliquée dans le phénomène de canitie précoce. La présente invention concerne donc également des procédés de criblage de molécules modulant l'interaction entre un polypeptide BNIPXL-beta et un polypeptide issu de la traduction du gène DDX31 permettant l'identification d'un agent pour une utilisation à des fins cosmétiques ou thérapeutiques, dans le domaine de la pigmentation.

Les différentes utilisations cosmétiques ou thérapeutiques au sens de la présente invention ont déjà été détaillées, ainsi que ce qu'il faut entendre par le domaine de la pigmentation, c'est-à-dire principalement la pigmentation des phanères, de préférence celle des cheveux. Par modulation de l'interaction, on entend une modification des paramètres caractérisant cette interaction, et tout particulièrement l'affinité. Une modulation s'entend d'une variation significative d'un point de vue statistique.

Un polypeptide issu de la traduction du gène DDX31 correspond à la forme longue (1-851) ou courte (1-585) de DDX31. De préférence, il s'agit de la forme longue de DDX31.

Selon une mise en oeuvre préférée ladite modulation est une disruption de l'association entre les deux polypeptides, de préférence une disruption totale.

Selon une autre mise en oeuvre du criblage, la modulation qui est recherchée est une modification de la constante d'association entre les deux polypeptides. Comme précédemment, par modification de la constante d'association, on entend une variation significative de cette constante, d'un point de vue statistique.

De préférence, par la mise en oeuvre du procédé, il est possible d'identifier un agent qui soit un inhibiteur compétitif de l'association entre les deux polypeptides.

Par ce procédé, il est donc possible de mettre en évidence des agents qui modulent l'interaction entre BNIPXLβ et un polypeptide issu de la traduction du gène DDX31 par exemple en se liant à BNIPXLβ et en empêchant ainsi son interaction avec ledit polypeptide, ou bien en se liant au polypeptide issu de la traduction du gène DDX31 et en empêchant ainsi son interaction avec BNIPXLβ.

La présente invention couvre aussi les agents identifiés par le procédé précédemment décrit.

Un agent modulant l'interaction entre BNIPXL-beta et un polypeptide issu de la traduction du gène DDX31, tel que défini dans le cadre de cette invention, peut être envisagé dans une application cosmétique ou thérapeutique, de préférence dans le domaine de la pigmentation. Un tel agent peut être identifiable ou identifié par le procédé de criblage décrit plus haut.

La divulgation décrit également l'utilisation d'un agent modulant l'interaction entre BNIPXL-beta et un polypeptide issu de la traduction du gène DDX31, pour la fabrication d'un médicament pour une utilisation thérapeutique dans le domaine de la pigmentation.

Des agents préférés, capables de moduler ladite interaction, sont des anticorps ou des fragments d'anticorps, de préférence dirigés contre BNIPXLβ ou contre un polypeptide issu de la traduction du gène DDX31. De tels anticorps peuvent être monoclonaux ou polyclonaux ; de préférence, il s'agit d'anticorps monoclonaux.

Pour toutes les utilisations ou applications selon l'invention dans le domaine de la cosmétique, le polypeptide, la molécule, l'agent ou le fragment polynucléotidique dont il est fait usage, peut être conditionné sous différentes formes appropriées, seul ou en combinaison avec d'autres agents. En particulier, des formes préférées sont destinées à des applications locales et elles concernent les crèmes, les lotions, les gels, les émulsions, les pommades et les shampoings. D'autres formes sont aussi envisageables pour des utilisations selon l'invention, en particulier sous forme de pilules pour une administration orale.

Parmi les différentes visées cosmétiques dans le cadre de la présente invention, un domaine particulièrement préféré est celui de la pigmentation. La pigmentation peut être celle de la peau ou bien des phanères. Il peut s'agir de la couleur de la pigmentation comme de l'absence de pigmentation ; les problèmes touchant à la qualité et à l'intensité de la pigmentation sont aussi concernés par la présente invention.

En particulier, l'objet de l'invention se rapporte à l'utilisation d'au moins un polypeptide, molécule, agent ou fragment polynucléotidique tel que défini plus haut, pour prévenir et/ou limiter et/ou arrêter le développement de la canitie.

L'objet de l'invention se rapporte aussi à l'utilisation d'au moins un polypeptide, molécule, agent ou fragment polynucléotidique tel que défini plus haut pour favoriser la pigmentation naturelle des cheveux et/ou des poils gris ou blancs.

Un autre objet de la présente invention se rapporte à un procédé de traitement cosmétique de la canitie caractérisé en ce qu'on applique sur la zone à traiter une composition comprenant au moins un polypeptide, molécule, agent ou fragment polynucléotidique tel que défini plus haut.

L'invention se rapporte aussi à un procédé de traitement cosmétique destiné à favoriser la pigmentation naturelle des cheveux et/ou des poils gris ou blancs caractérisé en ce qu'on applique sur la zone à traiter une composition comprenant au moins un polypeptide, molécule, agent ou fragment polynucléotidique tel que défini plus haut.

Les zones à traiter peuvent être, par exemple et sans aucune limitation, le cuir chevelu, les sourcils, la moustache, la barbe et/ou toutes les zones pileuses du corps.

Plus particulièrement, les procédés de traitement de la canitie et de pigmentation naturelle des cheveux et/ou poils gris ou blancs consistent à appliquer une composition comprenant au moins un polypeptide, molécule, agent ou fragment polynucléotidique tel que défini plus haut.

Concernant les différentes utilisations ou applications thérapeutiques de l'invention, il est en effet à noter que les affections touchant le système de pigmentation, que ce soit celui de la peau ou celui des phanères, peuvent avoir de graves conséquences sur la santé des personnes atteintes. En effet, la pigmentation de la peau joue le rôle de barrière protectrice face aux agressions lumineuses en particulier, les personnes souffrant d'albinisme sont dépourvues de protection faces aux rayons du soleil qui constituent pour eux un danger important. D'autres affections mettant en jeu la pigmentation sont aussi concernées par la présente invention.

Dans le cadre des utilisations thérapeutiques et cosmétiques tendant à modifier une caractéristique de la pigmentation, il s'agit de préférence de la pigmentation de la peau. Selon d'autres cas envisagés par la présente invention, le type de pigmentation qui doit être modifiée concerne la pigmentation des phanères, en particulier les ongles ou les poils. Selon un cas particulier préféré de la présente invention, la pigmentation dont on cherche à modifier les caractéristiques est celle du système pileux en général et de la chevelure, moustache et sourcils en particulier. La présente invention permet de modifier le phénomène d'arrêt de la pigmentation des cheveux, c'est-à-dire la canitie, en particulier lorsque celle-ci survient de manière prématurée chez une personne et que l'on parle de canitie précoce.

Pour toutes les utilisations en thérapeutique, les produits actifs entrant dans la composition d'un médicament sont de préférence associés à des excipients pharmaceutiquement acceptables. Toutes les voies d'administration considérées comme acceptables peuvent être utilisées dans le cadre de l'invention, en particulier voie intradermique, intraveineuse, musculaire, orale, otique, nasale, optique. La formulation est de préférence adaptée à la voie d'administration choisie.

Les utilisations pour la fabrication d'un médicament selon l'invention peuvent faire entrer dans leur formulation d'autres principes actifs. De même, l'administration d'un médicament comme défini dans l'invention peut être combinée avec l'administration d'un autre médicament, que cette administration soit simultanée, séquentielle ou séparée.

De même, les différents polypeptides, molécules, agents ou fragments polynucléotidiques tels que définis plus haut dont il est fait usage dans le cadre des utilisations en thérapie, peuvent être combinés et entrer dans la composition d'un unique médicament ou bien peuvent servir à la fabrication de différents médicaments. En particulier, s'ils entrent dans la composition de médicaments distincts, ils peuvent être administrés à des fréquences différentes, et/ou à des moments distincts.

De manière particulièrement préférée, les applications thérapeutiques ou cosmétiques envisagées dans le cadre cette invention concernent la prévention ou le traitement de la canitie et tout particulièrement la canitie précoce.

La présente invention concerne également des procédés de diagnostic d'une prédisposition à la canitie précoce. En effet, la canitie précoce est un phénotype qui a été défini par les inventeurs comme étant, entre autres, caractérisé par l'apparition des premiers cheveux blancs tôt dans la vie, et de préférence vers l'âge de 18 ou 20 ans. Comme ce phénotype est transmis à la génération suivante, il peut s'avérer important, pour les individus dont un parent ou un proche est atteint, de déterminer, avant l'apparition des symptômes, s'ils seront ou non sujets à cette atteinte. Le procédé de diagnostic selon l'invention est parfaitement adapté aux individus âgés de moins de 18 ans, voire moins de 20 ou 25 ans.

Comme il est très probable que des facteurs environnementaux jouent un rôle dans le phénotype « canitie » comme dans celui « canitie précoce », il s'agit, grâce aux procédés de l'invention, d'évaluer les risques de développer un tel phénotype, c'est-à-dire une prédisposition à la canitie précoce héréditaire.

En particulier, l'invention est dirigée vers un procédé pour le diagnostic d'une prédisposition à la canitie précoce chez un individu, comprenant les étapes suivantes :
- dosage du niveau d'expression du transcrit BNIPXL-beta à partir d'un échantillon corporel provenant dudit individu, et
- comparaison à un niveau de référence.

L'échantillon corporel peut être le sang, une unique goutte étant suffisante pour la mise en oeuvre d'un procédé selon l'invention. D'autres échantillons corporels peuvent être utilisés dans le cadre de l'invention tels que des cheveux, des poils, de l'urine ou de la sueur. Il est aussi envisageable d'utiliser quelques cellules issues de l'individu. L'homme de l'art saura déterminer quel échantillon pourra être utilisé dans le cadre de ce test, tout en minimisant le désagrément pour l'individu le subissant. De préférence, l'échantillon corporel utilisé dans le procédé de diagnostic décrit comprend ou est constitué de cellules mélanocytaires.

Les procédés de l'invention ne sont pas limités aux deux étapes décrites et peuvent contenir une ou plusieurs autres étapes antérieures, postérieures et/ou intermédiaires aux deux étapes mentionnées.

Le niveau de référence est une valeur reflétant le niveau d'expression du transcrit BNIPXL-beta chez des individus notoirement exempts d'une prédisposition à la canitie précoce. Inversement, il est bien entendu possible de définir comme niveau de référence un niveau reflétant le niveau d'expression du transcrit BNIPXL-beta chez des individus notoirement atteints de canitie précoce ou bien prédisposés à une telle affection.

### LEGENDE DES FIGURES:

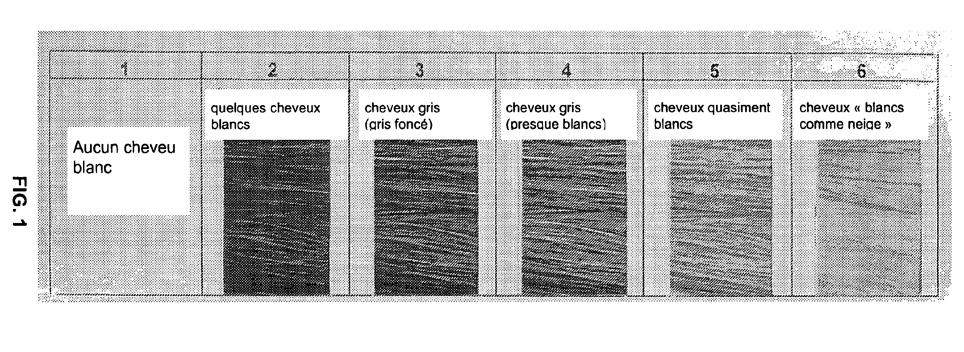

**FIG.1** : paramètres cliniques pour l'attribution du phénotype canitie précoce aux individus testés.
La figure 1 illustre l'échelle de référence pour l'index clinique canitique.
**FIG.2** (2A et 2B) : séquences relatives à la protéine BNIPXLβ.
La figure 2A illustre la séquence en acides aminés de la protéine BNIPXLβ, correspondant à SEQ ID N°1.
La figure 2B illustre la séquence nucléotidique du transcrit correspondant (ADNc) correspondant à SEQ ID N°2 ; la partie strictement codante est mise en évidence en majuscule (codant de 163 à 2361).

### SECTION EXPERIMENTALE:

Signification des acronymes utilisés :
CNC : région conservée non codante (« conserved non coding »),
IVS : région intronique (« intervening sequence »),
SNP : polymorphisme d'un unique nucléotide (« single nucleotide polymorphism »),

### Introduction

La canitie chez l'homme est un trait physique courant fortement lié au vieillissement. Dans la population européenne, la canitie commence généralement vers l'âge de 45 à 55 ans, en débutant par les tempes.

Il a déjà été fait état de syndromes monogéniques chez l'homme avec un processus de blanchiment prématuré du cheveu (ou canitie précoce) de manière localisée. Le piébaldisme par exemple, qui est un trait associé au syndrome de Waardenburg, se caractérise par un blanchiment de la zone frontale de manière prématurée, et il a été associé avec les gènes *PAX3* et *MITF* sur les chromosomes 3 et 2 respectivement (Baldwin et al, 1994 et Tassabehji et al, 1994).

De plus, il a été fréquemment observé que le blanchiment précoce des cheveux avait un caractère héréditaire. Le début de la canitie se situe dans l'enfance ; il survient avant l'âge de 25 ans et dans certains cas avant 18 ans. La canitie précoce a été décrite comme étant associée à certaines affections autoimmunes telle que l'anémie de Biermer ou la thyroïdite. Les présents inventeurs ont identifié des allèles liés à un risque de prédisposition à la canitie précoce (CP). A l'aide d'analyses de liaison et d'association pour des échantillons de cohortes de familles sélectionnées et de contrôles, ils ont pu identifier une variabilité au sein de la région chromosomique comprenant les gènes DDX31 et GTF3C4, sur le chromosome 9q. De plus, à l'aide d'analyse double-hybride en levure, les inventeurs ont également pu mettre en évidence l'implication fonctionnelle de la protéine DDX31 dans la cascade responsable de la pigmentation des phanères et celle de la protéine BNIPXL-β.

### RESULTATS

### Détermination du phénotype.

Dans cette étude, le phénotype « canitie précoce » (CP) fut attribué aux seuls individus (i) qui présentaient des cheveux blancs avant 25 ans et (ii) dont la moitié de la chevelure était grise à 30 ans et qui (iii) présentaient des antécédents familiaux de CP.

Ces caractéristiques ont été évaluées selon une échelle clinique afin d'obtenir, pour chaque individu, un score phénotypique global (fort pour des scores de 5 et 4, moyen pour des scores de 3 et 2, et faible pour un score de 1 ; voir Fig. 1 définissant l'index de canitie précoce et tableau 1).

**Tableau 1 : Méthode d'attribution du score clinique pour la canitie précoce.**

| | | |
|---|---|---|
| Pour chaque individu, les trois paramètres sont déterminés. Le score phénotypique global est obtenu en faisant la somme de chacun des paramètres. | | |

| Paramètres cliniques | | score |
|---|---|---|
| Début du blanchiment | Avant 18 ans | 2 |
| | Avant 25 ans | 1 |
| Blanchiment significatif de l'ensemble de la | Index de canitie 3 | 2 |
| chevelure à 35 ans | Index de canitie 2 | 1 |
| Caractère héréditaire | oui | 1 |
| | non | 0 |

### Analyse de liaison sur 12 familles :

Pour l'étude de liaison sur le génome global à l'aide de marqueurs microsatellites, 12 familles ont été retenues comprenant 96 individus (30 atteints de CP, 55 non atteints et 11 avec des phénotypes incertains) sur la base de calculs de puissance à partir de leurs pedigrees. Ces travaux sont plus particulièrement décrits dans les demandes WO04/007742 et FR2 865 217.

Sur le chromosome 9, les inventeurs ont déterminé un intervalle de liaison à la position 9q31-qter, entre les marqueurs D9S290 et D9S158, avec un lod score maximal à la position 151cM pour le marqueur D9S158.

### Etude d'association.

Une étude d'association (atteints par rapport à contrôle) a été réalisée sur l'ADN d'un groupe d'individus atteints de CP (scores 3-5), et sans lien entre eux et d'un groupe de contrôles. Cette étude a permis de mettre en évidence 33 SNPs sur le chromosome 9 (des SNPs rs2096071 à rs1378955) qui montrent une fréquence allélique différente entre les individus atteints de CP et les individus contrôles (test du Chi-2, p<0.05).

L'étape suivante a été le génotypage individuel des SNPs positifs sur le chromosome 9 dont la fréquence allélique différait sensiblement entre les atteints et les contrôles. Les inventeurs ont ainsi détecté 4 SNPs ayant des valeurs p significatives (voir tableau 2).

**Tableau 2: SNPs du chromosome 9q34 montrant une association significative avec la canitie précoce.**

| Nom du SNP | Valeur p |
|---|---|
| rs306534 | 4.25 x 10⁻³ |
| rs3739902 | 6 x 10⁻⁵ |
| rs575916 | 3.5 x 10⁻³ |
| rs365297 | 2 x 10⁻³ |

Par ailleurs, l'haplotype « B86-92 » (rs418320 et rs25260008) montre une association significative (p=0.0057) avec le trait CP. Cette région « B86-92 » a été réduite avec l'addition de 35 SNPs supplémentaires. Le SNP rs3739902, localisé dans l'intron 3 du gène DDX31 (IVS3+268), s'avère le plus significatif (p=6x10⁻⁵).

L'haplotype HAP86-88 est également particulièrement significatif, il est défini par les SNPs : rs3739902, rs2583805 et rs377090. La valeur statistique p d'association de cet haplotype avec la canitie précoce est inférieure à 10⁻⁶.

Un bloc important en déséquilibre de liaison a été observé par les inventeurs sur l'ensemble de la région comprenant les SNPs positifs. Deux gènes _DDX31, un membre de la famille protéique des hélicases à motif DEAD et GTF3C4, un facteur de transcription potentiel_ se trouvent au sein de ce bloc en déséquilibre de liaison.

Ces travaux sont plus amplement décrits dans les demandes WO04/007742 et FR2 865 217.

### Analyse de séquence des gènes candidats et des régions conservées non codantes dans la région comprise dans l'haplotype HAP86-88.

Les exons, les sites d'épissage, les régions 5' non traduites (5' UTR) et les régions conservées non codantes dans la région de l'haplotype HAP86-88 ont été séquencés pour une sélection d'ADN d'individus atteints. Six variants au niveau de la région codante et quatre variants introniques proches d'un site d'épissage ont été identifiés chez les individus atteints de canitie précoce (voir tableau 3).

**Tableau 3 : Variants identifiés lors de l'analyse de séquence des gènes DDX31 et GTF3C4, chez 12 individus atteints de canitie précoce.**

| gène | localisation | Exon/Intron | variant ADN | Signification fonctionnelle |
|---|---|---|---|---|
| **DDX31** | exon | 2 | c.413G>A | synonyme |
| | intron | 3 | c.723+15G>C | signification inconnue |
| | intron | 4 | c.767+15_17delCTC | signification inconnue |
| | intron | 4 | c.767+55C>T | synonyme, SNP rs4498679 |
| | intron | 11 | c.1176-16_13 delCTTA | signification inconnue |
| | exon | 13 | c.1674C>T | Synonyme, SNP rs306537 |
| | exon | 20 | c.2398G>A | p.Ala800Thr |
| | exon | 20 | c.2395A>G | p.lle799Val, SNP rs306547 |
| **GTF3C4** | exon | 1 | c.36G>A | synonyme |
| | exon | 3 | c.1560A>G | synonyme |

Le variant p.lle799Val (c.2395A>G, connu en tant que SNP rs306547) a été trouvé dans 6 des 12 ADN séquencés d'individus atteints à l'état hétérozygote et chez 6 atteints, à l'état homozygote (p.V799). Le 2^{nd} changement faux-sens p.Ala800Thr (c.2398G>A) a été trouvé à l'état hétérozygote chez un individu affecté. Afin d'estimer l'effet potentiel de ce variant, les inventeurs ont analysé une population plus importante d'individus atteints (62) et contrôles (64). Aucun autre porteur de cette mutation n'a été trouvé, ni dans les personnes atteintes de CP, ni dans les contrôles. Du fait que le résidu A800 n'a pas été conservé au cours de l'évolution des mammifères, car le résidu homologue chez la souris est la thréonine, comme dans le variant p.A800T, au lieu de l'alanine dans le gène humain, cet évènement est très probablement silencieux d'un point de vue biologique.

Un autre variant détecté est la délétion de CTC dans le motif constitué de la répétition CTCCTC dans l'intron 4 du gène DDX31 (IVS4+15_17deICTC).

Aucun variant n'a été détecté dans la séquence intergénique située dans les deux régions 5'UTR (régions non traduites) des gènes GTF3C4 et DDX31, qui sont orientés « tête bêche » par référence aux codons ATG.

Dans le gène GTF3C4, les inventeurs ont identifié 2 variants exoniques (exons 2 et 3).

Les inventeurs ont également analysé 20 séquences conservées et non codantes (CNC) au sein de ce locus. Parmi elles, un unique variant a été identifié, transistion c.2141-2018C>T, aussi connu comme SNP rs509762, situé dans l'intron 18 du gène DDX31 (position 134479481, NCBI build126). La comparaison des fréquences génotypique et allélique a montré que le génotype CP était surreprésenté chez les individus atteints avec un score de 5 (45% chez les atteints de canitie précoce par rapport à 32% pour les contrôles). Cette région conservée non codante CNC est conservée chez la souris, le poulet, et le fugu.

### Double hybride en levure

Afin d'investiguer un peu plus l'implication biologique de DDX31 comme gène de la canitie précoce, et de GTF3C4, les inventeurs ont utilisé la technologie dite du « double hybride » (ou deux hybrides) en levure afin de déterminer les partenaires potentiels d'interaction avec les isoformes tels que transcrits de ces gènes.

Brièvement les ADNc des formes longue (variant 1 complet, acides aminés 1-851) et courte (variant 2, tronqué dans la partie C-terminale, acides aminés 1-585) de DDX31 ont été amplifiés par réaction de polymérisation en chaîne à partir d'ARNm de mélanocyte humain et ont été clonés dans deux types de vecteurs, soit dans pB27 (LexA, fusion en C-terminal) et pB6 (Gal4, fusion en C-terminal). Le variant 2 de DDX31 code pour un polypeptide de 585 acides aminés qui est une forme tronquée dans la région C-terminale codée par les exons 16 à 20 du variant 1.

Ces constructions ont été utilisées comme appâts pour cribler une librairie de proies (10⁷ clones indépendants) spécifiques du mélanocyte humain. L'ADNc unique de GTF3C4 a également été utilisé comme appât, dans les mêmes conditions.

Le clonage des appâts, la construction de la librairie et l'établissement des cartes d'interaction ont été confiés à la société Hybrigenics (Paris, France). L'analyse des interactions a été menée à l'aide du logiciel PMRider® selon les procédures décrites dans Rain J.C. et al., 2001.

Pour les variants 1 et 2 de DDX31, respectivement 1,90.10⁶ et 38.10⁶ interactions ont été testées avec les construits pB27 et 45.10⁶ et 119.10⁶ interactions ont été testées avec les construits pB6.

Parmi les 21 protéines interagissant potentiellement avec le variant 1 de DDX31 et les 15 protéines interagissant potentiellement avec le variant 2 de DDX31, PAX3 a été identifié comme une protéine susceptible d'interagir avec DDX31, avec un score biologique prédit dans la classe « E » (PBS® « predicted biological score » défini et déterminé par le logiciel PMRider®). Cette interaction a été identifiée avec les deux formes, longue et courte, de DDX31. Cette interaction est centrée sur le domaine dit « homeobox » DEAD de la protéine PAX3. Il est à noter qu'il est connu que des mutations dans la protéine PAX3 sont associées au syndrôme de Waardenburg de type 1.

Pour GTF3C4 également une interaction significative avec le gène PAX3 a été identifiée. De façon surprenante et inattendue, les inventeurs ont également mis en évidence que la protéine BNIPXL-beta (BC12/adenovirus E1B 19kDa protein-interacting protein 2) était une proie de la forme longue de DDX31 (variant 1). Le score de confiance tel que défini par le programme était « C » (bonne confiance dans l'interaction), avec le variant 1 de DDX31. Les résultats sont présentés dans le tableau 4.

**Tableau 4 : (*) d'après Rain et al. , 2001.**

| **Nom de la protéine (appât)** | **Longueur de l'appât (AA)** | **Nom de la protéine (proie)** | **Score biologique global PIM® (*)** | **Activité biologique** |
|---|---|---|---|---|
| DDX31 Forme longue (exons 1-20) NM 022779 | 1-852 | PAX3 | E | Facteur de transciption gène 3 domaine paired Mutations associées au syndrome de Waardenburq de type 1. |
| DDX31 Forme courte (exons 1-15) NM 138620 | 1-585 | PAX3 | E | idem |
| DDX31 Forme longue (exons 1-20) NM_022779 | 1-852 | BNIPXLβ | C | Protéine interagissant avec Bcl2 et la protéine de 19kDa de l'adénovirus E1B. Impliquée dans la suppression de la mort cellulaire |

### DISCUSSION

La canitie précoce représente un état clinique particulièrement informatif dans la compréhension des bases génétiques liées au blanchiment des cheveux, un trait généralement associé à la vieillesse.

Suite à une étude d'association, les inventeurs ont mis en évidence un locus de liaison à la CP sur le chromosome 9. Des analyses d'association avec des SNPs a permis de réduire l'intervalle de liaison d'initialement 10 Mpb à une région beaucoup plus petite d'environ 100kpb. Cette région est organisée comme un unique bloc en déséquilibre de liaison qui héberge deux gènes : DDX31 et GTF3C4.

En considérant que la canitie précoce pourrait être un trait probablement multigénique, et que ce locus a été obtenu alors même que le nombre d'individus atteints analysés est limité, on peut en conclure que selon toute vraisemblance, ce locus est susceptible de jouer une rôle majeur dans la canitie précoce.

Il faut noter que les résultats de l'approche double hybride ont révélé que BNIPXLβ était une proie commune des isoformes alternatifs long et court de DDX31. BNIPXLβ est de plus un facteur impliqué dans le processus d'apoptose (Vegeto et al, 1999).

Le séquençage des séquences codantes, d'épissage et conservées non codantes (CNC) de l'ADN de 12 individus atteints de CP avec les phénotypes les plus rigoureux, a permis d'identifier plusieurs variants. Dans DDX31, le variant p.Ala800Thr dans l'exon 20, non répertorié comme polymorphisme, pourrait être à l'origine d'un effet fonctionnel lié à la canitie précoce. La variation IVS4+15_17deICTC se situe dans une région non conservée parmi les mammifères et il n'a pas été prédit que cette région puisse altérer l'épissage de l'intron 4 (analyse dite « splice-view »). Ce variant pourrait être associé avec la variation génétique responsable du trait phénotypique.

La variation au sein de CNChs8, dans l'intron 18 du gène DDX31 présente un meilleur potentiel phénotypique du fait qu'il se situe dans une portion de séquences qui s'avèrent très conservées chez la souris (pas d'espace, pas de changement sur une distance de 100 paires de bases). De manière intéressante, l'approche double-hybride a permis de démontrer que DDX31 et GTF3C4, tous deux situés dans la région impliquée dans la canitie précoce, ont une interaction commune avec PAX3, protéine soupçonnée de longue date de faire partie du processus de pigmentation.

Il est également à noter que DDX31 code pour une hélicase. Dans le syndrome de Werner, correspondant à un état de vieillissement accéléré pour lequel un blanchiment prématuré des cheveux est observé, il a été rapporté que le gène responsable code pour une hélicase (WRN) (Yu et al, 1996).

De façon surprenante et inattendue, les inventeurs ont découvert que BNIPXL-beta (BCI2/adenovirus E1B 19kDa protein-interacting protein 2) était une proie de DDX31. Le score de confiance dans l'interaction est particulièrement bon.

L'interaction entre DDX31 et BNIPXL-beta est donc probablement importante dans le trafic vésiculaire, d'où un rôle dans la migration et le transfert des mélanosomes et donc la canitie et la canitie précoce.

### MATERIEL ET METHODES

### Individus atteints, Etude de liaison et Etude d'association

Le choix des individus et leur classification ont été réalisés comme indiqué dans les demandes FR2842104, FR2853532, FR2865217 et WO04/007742.

Les études de liaison et d'association ont également été décrites dans ces demandes de brevet ainsi que dans les abrégés Blouin *et al,* 2006 et de Lacharrière *et al,* 2007.

Pour rappel, dans une première étape, les inventeurs ont génotypé des échantillons regroupant les ADN des individus atteints par rapport aux contrôles appariés, pour une sélection de SNPs compris dans l'intervalle du chromosome 9 montrant une liaison significative avec le trait canitie précoce.

La région correspondant à l'intervalle de liaison sur le chromosome 9q34 a été défini entre les marqueurs SNPs rs2096071 et rs1099298 (Entre les positions 123'405'258 bp et 132'547'291 bp, exprimées en fonction de la version de 2001 (soit NCBI Build28) ; entre les position 130'565'549 et 139'556'369 selon la version 37 ; genome.cse.uscsc.edu).

Un SNP donné est considéré comme « positif » s'il montre une déviation significative dans la fréquence des allèles. Chaque région qui remplit au moins l'une des conditions suivantes : (i) au moins 2 SNPs positifs contigus et (ii) 2 SNPs positifs séparés par un unique SNP négatif ; est sélectionnée pour la phase de génotypage individuel.

Afin de tester l'association des SNPs sélectionnés avec le phénotype CP dans chaque ADN génotypé individuellement (phase 2 de l'analyse), les inventeurs ont comparé la fréquence allélique or la fréquence génotypique de chaque SNP dans les populations d'atteints et de contrôles.

Afin de saisir toute la variation génétique de cette région, les inventeurs ont également procédé à une analyse d'haplotype, à l'aide d'une fenêtre mobile (comprenant 5 SNPs à chaque fois, avec un incrément de 1), afin de reconstruire efficacement l'haplotype.

### Séquençage :

L'ADN d'individus atteints de CP a été amplifié au niveau des exons des gènes GTF3C4 et DDX31, de la petite région intergénique entre ces gènes, et au niveau des régions comprenant des séquences CNC présentant au moins 70% d'identité entre l'homme et la souris (sans espace), et séquencé par des méthodes classiques.

### Double-hybride :

Le clonage des appâts, la construction de la librairie et l'établissement des cartes d'interaction ont été confiés à la société Hybrigenics (Paris, France). L'analyse des interactions a été menée à l'aide du logiciel PMRider® selon les procédures décrites dans Rain J.C. et al., 2001. Les variants d'épissage long et court de DDX31 (numéros d'accession GenBank NM_022779 et NM_138620, respectivement) la protéine STX17 humaine (numéro d'accession GenBank NM_017919) ont été clonés dans pB27 en phase avec LexA et dans pB6 en phase avec le domaine de liaison à l'ADN de Gal4. Les quatre construits ont été utilisés comme appât pour cribler une librairie d'ADNc spécifiques du mélanocyte humain, construite dans pP6. Les plasmides pB27, pB6 et pP6 sont dérivés des plasmides pBTM116 (Vojtek and Hollenberg, 1995), pAS2ΔΔ (Fromont-Racine et al., 1997) et pGADGH (Bartel et al, 1993), respectivement.

Une moyenne de 73 millions de clones (7 fois la complexité de la librairie) a été criblée pour chaque appât, en utilisant une approche de croisement avec des souches de levures L40-Gal4 (mat a) et Y187 (mat α) comme précédemment décrit dans (Fromont-Racine et al., 1997). Des colonies His+ ont été sélectionnées sur un milieu dépourvu de tryptophane, de leucine et d'histidine. Les fragments de proies des clones positifs ont été amplifiés par PCR et séquencés à leurs jonctions 3' et 5' sur un séquenceur PE3700. Les séquences résultantes ont été utilisées pour identifier les protéines interagissant correspondantes dans la banque de données GenBank (NCBI), en utilisant un procédé entièrement automatisé. Un score de confiance a été attribué à chaque interaction de la manière déjà décrite (Formstecher et al, 2005).

Le score local est la probabilité pour un domaine d'interaction donné (« Selected interacting domain », SID®) d'être obtenu en faisant l'hypothèse de l'égalité des chances, c'est-à-dire du fait du bruit aléatoire. On peut le déduire en combinant les probabilités p (en utilisant une loi binomiale) de chacun des fragments indépendants le définissant. Un score (global) biologique prédit (« PBS ») est calculé pour chacune des protéines d'interaction après mise en commun des résultats de chaque criblage. En faisant l'hypothèse d'indépendance des évènements, les scores des différents criblages sont combinés ensemble quand la même paire de domaines protéiques est concernée. Le PBS résultant représente donc la probabilité qu'une interaction protéine-protéine donnée soit due au bruit.

Les scores sont des nombres réels, compris entre 0 et 1, mais en pratique ils sont regroupés en quatre catégories (classes A, B, C et D). Enfin, la connectivité globale de la carte d'interaction est analysée pour étiqueter séparément (catégorie E) les SID (domaine d'interaction sélectionné) trouvés comme proie à une fréquence supérieure à un seuil fixé : le PBS de chaque interaction protéine-protéine impliquant des SID très fortement liés est fixé à 1. A la fois les seuils entre les catégories et le seuil de forte liaison sont définis manuellement, en tenant compte de la nature de l'organisme étudié, de la librairie utilisée et de la présente couverture du protéome (A<1^{e}610<B<1^{e}-5<C<1^{e}-2,5<D ; la catégorie E correspond aux SID proies sélectionnées avec plus de 4 appâts et a été attribué de manière arbitraire la valeur 1.

La présente demande concerne plus particulièrement les points ou objets suivants :
A. un polypeptide comprenant une séquence présentant au moins 90% d'identité avec tout ou partie de la séquence SEQ ID N°1, pour une utilisation thérapeutique dans le traitement ou la prévention de la canitie précoce chez l'homme, ladite partie comprenant au moins 30 acides aminés ;
B. une molécule comprenant une séquence d'ARNi ayant au moins 90% d'identité avec tout ou partie de la séquence SEQ ID N°2, pour une utilisation thérapeutique dans le traitement ou la prévention de la canitie précoce chez l'homme, ladite partie comprenant au moins 18 nucléotides ;
C. un procédé de criblage de molécules modulant l'expression du gène codant pour BNIPXL-beta afin d'identifier un agent pour une utilisation à des fins cosmétiques ou thérapeutiques, dans le domaine de la pigmentation ;

Le procédé de criblage (C) peut comprendre les étapes suivantes :
- mise en présence de la molécule à tester et du gène codant pour BNIPXL-beta, dans des conditions permettant l'expression dudit gène en l'absence de la molécule à tester et
- détection d'une variation dans le niveau d'expression dudit gène, du fait de la présence de la molécule à tester.

Dans le procédé de criblage (C), la modulation de l'expression peut être une inhibition de la transcription ou de la traduction du gène codant pour BNIPXL-beta.

Dans le procédé de criblage (C), les molécules à tester peuvent être des protéines, des ARNi, des ribozymes ou des ARN antisens ciblant le gène codant pour BNIPXL-beta.
D. Un procédé de criblage de molécules modulant l'activité du polypeptide BNIPXL-beta, pour l'identification d'un agent pour une utilisation à des fins cosmétiques ou thérapeutiques, dans le domaine de la pigmentation ;

Le procédé de criblage (D) peut comprendre les étapes suivantes :
- mise en présence de la molécule à tester et du polypeptide BNIPXL-beta, dans des conditions permettant la mise en évidence de l'activité dudit polypeptide en l'absence de la molécule à tester et
- détection d'une variation dans l'activité dudit polypeptide, du fait de la présence de la molécule à tester.

Dans le procédé de criblage (D), la modulation de l'activité peut être une inhibition, totale ou partielle.

Dans le procédé de criblage (D), la modulation de l'activité peut être réalisée par séquestration dudit polypeptide.

Dans le procédé de criblage (D), les molécules à tester peuvent être des anticorps dirigés contre le polypeptide BNIPXL-beta ou une partie de celui-ci.

Dans les procédés de criblage (C) et (D), la pigmentation est celle des cheveux.
E. L'utilisation d'un agent modulant l'expression du gène codant pour BNIPXL-beta, à des fins cosmétiques, dans le domaine de la pigmentation.
F. L'utilisation d'un agent modulant l'expression du gène codant pour BNIPXL-beta, pour la fabrication d'un médicament pour une utilisation thérapeutique dans le domaine de la pigmentation.
1. L'utilisation d'au moins un fragment polynucléotidique comprenant au moins 18 nucléotides consécutifs dont la séquence correspond à tout ou partie du gène codant pour BNIPXL-beta ou du transcrit BNIPXL-beta, à des fins cosmétiques, dans le domaine de la pigmentation.

Les utilisations (E), (F), et (I) concernent la prévention ou le traitement de la canitie, notamment la canitie précoce.
J. Un procédé pour le diagnostic d'une prédisposition à la canitie précoce chez un individu, comprenant les étapes suivantes :
   i) Dosage du niveau d'expression du transcrit BNIPXL-beta à partir d'un échantillon corporel provenant dudit individu,
   ii) Comparaison à un niveau de référence.

Dans le procédé (J), le niveau de référence est le niveau d'expression du transcrit BNIPXL-beta chez un individu non atteint.

Dans le procédé (J), l'échantillon corporel peut comprendre ou être constitué de cellules mélanocytaires.
K. Un procédé de criblage de molécules modulant l'interaction entre un polypeptide BNIPXL-beta et un polypeptide issu de la traduction du gène DDX31 permettant l'identification d'un agent pour une utilisation à des fins cosmétiques ou thérapeutiques, dans le domaine de la pigmentation.

Dans le procédé de criblage (K), la modulation peut être une disruption de l'association entre les deux polypeptides.

Dans le procédé de criblage (K), la modulation peut être une modification de la constante d'association entre les deux polypeptides.

Dans le procédé de criblage (K), l'agent identifié peut être un inhibiteur compétitif de l'association entre les deux polypeptides.

Dans le procédé de criblage (K), la pigmentation est celle des cheveux.

### Références

Belcredito, S., et al. 2001. Estrogen neuroprotection: the involvement of the Bcl-2 binding protein BNIP2. Brain Res Brain Res Rev 37: 335-342.
Blouin, JL et al. Localisation of a gene for Human Premature Hair Greying on chromosome 9q34. Congress of the American Society of Human Genetics, october 2006.
Commo, S. & Bernard, B.A. 2000. Melanocyte subpopulation turnover during the human hair cycle : an immunohistochemical study. Pigment Cell Res. 13 :253-259
Commo S ., Gaillard O., Thibaut S., & Bernard B.A. 2004. Absence of TRP-2 in melanogenic melanocytes of human hair. Pigment Cell Res. 17:488-497
Commo, S., Gaillard, O. & Bernard, B. A. Human hair greying is linked to a specific depletion of hair follicle melanocytes affecting both the bulb and the outer root sheath. Br J Dermatol 150, 435-43 (2004).
Formstecher, E. et al. Protein interaction mapping: a Drosophila case study. Genome Res 15, 376-84 (2005).
Fromont-Racine, M., Rain, J. C. & Legrain, P. Toward a functional analysis of the yeast genome through exhaustive two-hybrid screens. Nat Genet 16, 277-82 (1997).
**de** Lacharrière O., et al. A gene for premature hair greying maps to chromosome 9q34. World Congress of Dermatology, october 2007.
Machida, T., et al. 2006. Increased expression of proapoptotic BMCC1, a novel gene with the BNIP2 and Cdc42GAP homology (BCH) domain, is associated with favorable prognosis in human neuroblastomas. Oncogene 25: 1931-1942.
Mousley, C. J., et al. 2007. The Sec14-superfamily and the regulatory interface between phospholipid metabolism and membrane trafficking. Biochim Biophys Acta 1771: 727-736.
Qin, W., et al. 2003. BNIPL-2, a novel homologue of BNIP-2, interacts with Bcl-2 and Cdc42GAP in apoptosis. Biochem Biophys Res Commun 308: 379-385.
Rain, J. C. et al. The protein-protein interaction map of Helicobacter pylori. Nature 409, 211-5 (2001).
Shang, X., et al. 2003. Concerted regulation of cell dynamics by BNIP-2 and Cdc42GAP homology/Sec14p-like, proline-rich, and GTPase-activating protein domains of a novel Rho GTPase-activating protein, BPGAP1. J Biol Chem 278: 45903-45914.
Sirokmany, G., et al. 2006. Sec14 homology domain targets p50RhoGAP to endosomes and provides a link between Rab and Rho GTPases. J Biol Chem 281: 6096-6105.
Tassabehji, M., Newton, V. E. & Read, A. P. Waardenburg syndrome type 2 caused by mutations in the human microphthalmia (MITF) gene. Nat Genet 8, 251-5 (1994).
Vegeto, E., et al Estrogen and progesterone induction of survival of monoblastoid cells undergoing TNF-alpha-induced apoptosis. Faseb J 13, 793-803 (1999).
Vojtek, A. B. & Hollenberg, S. M. Ras-Raf interaction: two-hybrid analysis. Methods Enzymol 255, 331-42 (1995).
Yu, C. E. et al. Positional cloning of the Werner's syndrome gene. Science 272, 258-62 (1996).
Zhou, Y. T., Guy, G. R. & Low, B. C. 2005. BNIP-2 induces cell elongation and membrane protrusions by interacting with Cdc42 via a unique Cdc42-binding motif within its BNIP-2 and Cdc42GAP homology domain. Exp Cell Res 303: 263-274.

### SEQUENCE LISTING

<110> L'OREAL
<120> utilisations de BNIPXL-beta dans la canitie précoce
<130> B07637A - CA/CS
<150> FR 08/02435
   <151> 2008-04-30
<160> 3
<170> PatentIn version 3.3
<210> 1
   <211> 732
   <212> PRT
   <213> Homo sapiens
<400> 1
<210> 2
   <211> 2405
   <212> DNA
   <213> Homo sapiens
<220>
   <221> CDS
   <222> (163)..(2361)
<400> 2
   gctttgtttg atggtgatcc acatttatcc acagagaatc ctgccttggt tcctgatgct      60
   ttgctagcct cagacacttg tctggatata agcgaagctg cctttgacca cagtttcagc     120
   gatgcctcag gtctcaacac atccacggga acaatagatg ac atg agt aaa ctg        174
   aca tta tcc gaa ggc cat ccg gaa acg cca gtt gat ggg gac cta ggg       222
   aag caa gat atc tgc tca tct gaa gcc tcg tgg ggt gat ttt gaa tat       270
   gat gta atg ggc cag aat atc gat gaa gat tta ctg aga gag cct gaa       318
   cac ttc ctg tat ggt ggt gac cct cct ttg gag gaa gat tct ctg aag       366
   cag tcg ctg gca ccg tac aca cct ccc ttt gat ttg tct tat atc aca       414
   gaa cct gcc cag agt gct gaa aca ata gag gaa gct ggg tct cca gag       462
   gat gaa tct ctg gga tgc aga gca gca gag ata gtg ctt tct gca ctt       510
   cct gat cga aga agt gag gga aac cag gct gag acc aaa aac aga ctg       558
   cct gga tcc cag ctg gct gtg ctg cat att cgt gaa gac cct gag tcc       606
   gtt tat ttg ccg gta gga gca ggc tcc aac att ttg tct cca tca aac       654
   gtt gac tgg gaa gta gaa aca gat aat tct gat tta cca gca ggt gga       702
   gac ata gga cca cca aat ggt gcc agc aag gaa ata tca gaa ttg gaa       750
   gaa gaa aaa aca att cct acc aaa gag cct gag cag ata aaa tca gaa       798
   tac aag gaa gaa aga tgt aca gag aag aat gaa gat cgt cat gca cta      846
   cac atg gat tac ata ctt gta aac cgt gaa gaa aat tca cac tca aag      894
   cca gag acc tgt gaa gaa aga gaa agc ata gct gaa tta gaa ttg tat      942
   gta ggt tcc aaa gaa aca ggg ctg cag gga act cag tta gca agc ttc      990
   cca gac aca tgt cag cca gcc tcc tta aat gaa aga aaa ggt ctc tct     1038
   gca gag aaa atg tct tct aaa agc gat acg aga tca tct ttt gaa agc     1086
   cct gca caa gac cag agt tgg atg ttc ttg ggc cat agt gag gtt ggt     1134
   gat cca tca ctg gat gcc agg gac tca ggg cct ggg tgg tcc ggc aag     1182
   act gtg gag ccg ttc tct gaa ctc ggc ttg ggt gag ggt ccc cag ctg     1230
   cag att ctg gaa gaa atg aag cct cta gaa tct tta gca cta gag gaa     1278
   gcc tct ggt cca gtc agc caa tca cag aag agt aag agc cga ggc agg     1326
   gct ggc ccg gat gca gtt acc cat gac aat gaa tgg gaa atg ctt tca     1374
   cca cag cct gtt cag aaa aac atg atc cct gac acg gaa atg gag gag     1422
   gag aca gag ttc ctt gag ctc gga acc agg ata tca aga cca aat gga     1470
   cta ctg tca gag gat gta gga atg gac atc ccc ttt gaa gag ggc gtg     1518
   ctg agt ccc agt gct gca gac atg agg cct gaa cct cct aat tct ctg     1566
   gat ctt aat gac act cat cct cgg aga atc aag ctc aca gcc cca aat     1614
   atc aat ctt tct ctg gac caa agt gaa gga tct att ctc tct gat gat      1662
   aac ttg gac agc cca gat gaa att gac atc aat gtg gat gaa ctt gat      1710
   acc ccc gat gaa gca gat tct ttt gag tac act ggc cat gaa gat ccc      1758
   aca gcc acc aaa gat tct ggc caa gag tca gag tct att cca gaa tat      1806
   acg gcc gaa gag gaa cgg gag gac aac cgg ctt tgg agg aca gtg gtc      1854
   att gga gac caa gag cag cgc att gac atg aag gtc atc gag ccc tac      1902
   agg aga gtc att tct cac gga gga ctt aga gga tac tat ggg gac ggt      1950
   cta aat gcc atc att gtg ttt gcc gcc tgt ttt ctg cca gac agc agt      1998
   cgg gcg gat tac cac tat gtc atg gaa aat ctt ttc cta tat gta ata      2046
   agt act tta gag ttg atg gta gct gaa gac tat atg att gtg tac ttg      2094
   aat ggt gca acc cca aga agg agg atg cca ggg cta ggc tgg atg aag      2142
   aaa tgc tac cag atg att gac aga cgg ttg agg aag aat ttg aaa tca      2190
   ttc atc att gtt cat cca tct tgg ttc atc aga aca atc ctt gct gtg      2238
   aca cga cct ttt ata agt tca aaa ttc agc agt aaa att aaa tat gtc      2286
   aat agc tta tca gaa ctc agt ggg ctg atc cca atg gat tgc atc cac      2334
   att cca gag agc atc atc aaa tat tga cttgaagctg aaagaaaagc            2381
   cttagttggc catgctggaa gaag                                           2405
<210> 3
   <211> 732
   <212> PRT
   <213> Homo sapiens
<400> 3

## Revendications

1. Polypeptide comprenant une séquence présentant au moins 90% d'identité avec tout ou partie de la séquence SEQ ID N°1, pour une utilisation thérapeutique dans le traitement ou la prévention de la canitie précoce chez l'homme, ladite partie comprenant au moins 30 acides aminés.

2. Molécule comprenant une séquence d'ARNi ayant au moins 90% d'identité avec tout ou partie de la séquence SEQ ID N°2, pour une utilisation thérapeutique dans le traitement ou la prévention de la canitie précoce chez l'homme, ladite partie comprenant au moins 18 nucléotides.

3. Procédé de criblage de molécules modulant l'expression du gène codant pour le polypeptide BNIPXL-beta de séquence SEQ ID N°1 afin d'identifier un agent pour une utilisation à des fins cosmétiques ou thérapeutiques, dans le domaine de la pigmentation des cheveux, comprenant les étapes suivantes :
- mise en présence de la molécule à tester et du gène codant pour BNIPXL-beta, dans des conditions permettant l'expression dudit gène en l'absence de la molécule à tester et
- détection d'une variation dans le niveau d'expression dudit gène, du fait de la présence de la molécule à tester.

4. Procédé selon la revendication 3, où ladite modulation de l'expression est une inhibition de la transcription ou de la traduction du gène codant pour le polypeptide BNIPXL-betade séquence SEQ ID N°1.

5. Procédé selon l'une des revendications 3 à 4, où les molécules à tester sont des protéines, des ARNi, des ribozymes ou des ARN antisens ciblant le gène codant pour le polypeptide BNIPXL-betade séquence SEQ ID N°1.

6. Procédé de criblage de molécules modulant l'activité du polypeptide BNIPXL-beta de séquence SEQ ID N°1, pour l'identification d'un agent pour une utilisation à des fins cosmétiques ou thérapeutiques, dans le domaine de la pigmentation des cheveux, comprenant les étapes suivantes :
- mise en présence de la molécule à tester et du polypeptide BNIPXL-beta, dans des conditions permettant la mise en évidence de l'activité dudit polypeptide en l'absence de la molécule à tester et
- détection d'une variation dans l'activité dudit polypeptide, du fait de la présence de la molécule à tester.

7. Procédé selon la revendication 6, où les molécules à tester sont des anticorps dirigés contre le polypeptide BNIPXL-beta de séquence SEQ ID N°1 ou une partie de celui-ci.

8. Utilisation d'un agent modulant l'expression du gène codant pour le polypeptide BNIPXL-beta de séquence SEQ ID N°1, à des fins cosmétiques, dans le domaine de la pigmentation pour la prévention ou le traitement de la canitie, où ledit agent est une molécule selon la revendication 2.

9. Agent modulant l'expression du gène codant pour le polypeptide BNIPXL-betade séquence SEQ ID N°1, pour une utilisation thérapeutique dans le domaine de la pigmentation des cheveux, où ledit agent est une molécule selon la revendication 2.

10. Utilisation d'au moins un fragment polynucléotidique comprenant au moins 18 nucléotides consécutifs dont la séquence correspond à tout ou partie du gène codant pour le polypeptide BNIPXL-betade séquence SEQ ID N°1 ou du transcrit BNIPXL-betade séquence SEQ ID N°2, à des fins cosmétiques, dans le domaine de la pigmentation, pour la prévention ou le traitement de la canitie.

11. Procédé pour le diagnostic d'une prédisposition à la canitie précoce chez un individu, comprenant les étapes suivantes :
i) Dosage du niveau d'expression du transcrit BNIPXL-beta de séquence SEQ ID N°2 à partir d'un échantillon corporel provenant dudit individu,
ii) Comparaison à un niveau de référence, où le niveau de référence est le niveau d'expression du transcrit BNIPXL-beta chez un individu non atteint de canitie précoce.

12. Procédé de criblage de molécules modulant l'interaction entre un polypeptide BNIPXL-beta de séquence SEQ ID N°1 et un polypeptide issu de la traduction du gène DDX31 permettant l'identification d'un agent pour une utilisation à des fins cosmétiques ou thérapeutiques, dans le domaine de la pigmentation des cheveux.

13. Procédé selon la revendication 12, où ladite modulation est une modification de la constante d'association entre les deux polypeptides.

## Claims

1. A polypeptide comprising a sequence having at least 90% sequence identity with all or part of the sequence SEQ ID NO: 1, for therapeutic use in the treatment or prevention of premature canities in human, said part comprising at least 30 amino acids.

2. A molecule comprising a RNAi sequence having at least 90% sequence identity with all or part of the sequence SEQ ID NO: 2 for therapeutic use in the treatment or prevention of premature canities in human, said part comprising at least 18 nucleotides.

3. A method for screening molecules modulating the expression of a gene coding for the BNIPXL-beta polypeptide having the sequence SEQ ID N°1, in order to identify an agent for use for cosmetic or therapeutic purposes, in the field of hair pigmentation comprising the following steps:
• bringing a test molecule into the presence of a gene coding for BNIPXL-beta under conditions allowing expression of said gene in the absence of the test molecule; and
• detecting a variation in the level of expression of said gene due to the presence of the test molecule.

4. A method according to claim 3, wherein said modulation of expression is inhibition of transcription or translation of the gene coding for the BNIPXL-beta polypeptide having the sequence SEQ ID N°1.

5. A method according to claim 3 or 4, wherein the test molecules are proteins, RNAi's, ribozymes or antisense RNA targeting the gene coding for the BNIPXL-beta polypeptide having the sequence SEQ ID N°1.

6. A method for screening molecules modulating the activity of the BNIPXL-beta polypeptide having the sequence SEQ ID N°1, to identify an agent for use for cosmetic or therapeutic purposes, in the field of hair pigmentation, comprising the following steps:
• bringing the test molecule into the presence of the BNIPXL-beta polypeptide, under conditions allowing the detection of activity of said polypeptide in the absence of the test molecule; and
• detecting a variation in the activity of said polypeptide due to the presence of the test molecule.

7. A method according to claim 6, wherein the test molecules are antibodies directed against the BNIPXL-beta polypeptide having the sequence SEQ ID N°1 or a portion thereof.

8. Use of an agent modulating the expression of a gene coding for the BNIPXL-beta polypeptide having the sequence SEQ ID N°1, for cosmetic purposes, in the field of pigmentation, for the prevention or treatment of canities, wherein said agent is a molecule according to claim 2.

9. An agent modulating the expression of a gene coding for the BNIPXL-beta polypeptide having the sequence SEQ ID N°1, for therapeutic use, in the field of hair pigmentation, wherein said agent is a molecule according to claim 2.

10. Use of at least one polynucleotide fragment comprising at least 18 consecutive nucleotides the sequence of which corresponds to all or a portion of the gene coding for the BNIPXL-beta polypeptide having the sequence SEQ ID N°1 or a transcript of BNIPXL-beta having the sequence SEQ ID N°2, for cosmetic purposes, in the field of pigmentation, for the prevention or treatment of canities.

11. A method for diagnosing a predisposition to premature canities in an individual, comprising the following steps:
i) assay of the level of expression of a BNIPXL-beta transcript having the sequence SEQ ID N°2 using a body sample from said individual;
ii) comparison with a reference level, wherein the reference level is the level of expression of the BNIPXL-beta transcript in an individual unaffected by premature canities.

12. A method for screening molecules modulating the interaction between a BNIPXL-beta polypeptide having the sequence SEQ ID N°1 and a polypeptide derived from translation of the DDX31 gene, to identify an agent for use for cosmetic or therapeutic purposes in the field of hair pigmentation.

13. A method according to claim 12, wherein said modulation is a modification of the association constant between the two polypeptides.

## Patentansprüche

1. Polypeptid enthaltend eine Sequenz mit mindestens 90 % Identität zur vollständigen oder partiellen Sequenz von SEQ ID Nr. 1 für eine therapeutische Verwendung zur Behandlung von bzw. Vorbeugung vor vorzeitiger Haarergrauung beim Menschen, wobei die partielle Sequenz mindestens 30 Aminosäuren enthält.

2. Molekül enthaltend eine RNAi-Sequenz mit mindestens 90 % Identität zur vollständigen oder partiellen Sequenz von SEQ ID Nr. 2 für eine therapeutische Verwendung zur Behandlung von bzw. Vorbeugung vor vorzeitiger Haarergrauung beim Menschen, wobei die partielle Sequenz mindestens 18 Nukleotide enthält.

3. Verfahren zum Screening von Molekülen, die die Expression des für das Polypeptid BNIPXL-beta der Sequenz von SEQ ID Nr. 1 kodierenden Gens modulieren, zum Identifizieren eines Wirkstoffs für eine Verwendung zu kosmetischen oder therapeutischen Zwecken im Bereich der Haarpigmentierung, die folgenden Schritte umfassend:
- Gegenüberstellen des Testmoleküls und des für BNIPXL-beta kodierenden Gens unter Bedingungen, die die Expression des Gens bei Fehlen des Testmoleküls ermöglichen, und
- Ermitteln einer Variation auf der Expressionsebene des Gens infolge des Vorhandenseins des Testmoleküls.

4. Verfahren nach Anspruch 3,
bei dem die Modulation der Expression eine Hemmung der Transkription oder der Translation des für das Polypeptid BNIPXL-beta der Sequenz von SEQ ID Nr. 1 kodierenden Gens ist.

5. Verfahren nach einem der Ansprüche 3 bis 4,
bei dem die Testmoleküle Proteine, RNAis, Ribozyme oder Antisense-RNAs sind, die gegen das Polypeptid BNIPXL-beta der Sequenz von SEQ ID Nr. 1 kodierende Gen gerichtet sind.

6. Verfahren zum Screening von Molekülen, die die Aktivität des Polypeptids BNIPXL-beta der Sequenz von SEQ ID Nr. 1 modulieren, zum Identifizieren eines Wirkstoffs für eine Verwendung zu kosmetischen oder therapeutischen Zwecken im Bereich der Haarpigmentierung, die folgenden Schritte umfassend:
- Gegenüberstellen des Testmoleküls und des Polypetids BNIPXL-beta unter Bedingungen, die den Nachweis der Aktivität des Polypeptids bei Fehlen des Testmoleküls ermöglichen, und
- Ermitteln einer Variation in der Aktivität des Polypeptids infolge des Vorhandenseins des Testmoleküls.

7. Verfahren nach Anspruch 6,
bei dem die Testmoleküle Antikörper sind, die gegen das Polypeptid BNIPXL-beta der Sequenz von SEQ ID Nr. 1 oder einem Teil von diesem gerichtet sind.

8. Verwendung eines Wirkstoffs, der die Expression des das Polypeptid BNIPXL-beta der Sequenz von SEQ ID Nr. 1 kodierenden Gens moduliert, zu kosmetischen Zwecken im Bereich der Pigmentierung zur Vorbeugung vor bzw. Behandlung von Haarergrauung, wobei der Wirkstoff ein Molekül nach Anspruch 2 ist.

9. Wirkstoff, der die Expression des für das Polypeptid BNIPXL-beta der Sequenz von SEQ ID Nr. 1 kodierenden Gens moduliert, zu therapeutischen Zwecken im Bereich der Haarpigmentierung, wobei der Wirkstoff ein Molekül nach Anspruch 2 ist.

10. Verwendung von wenigstens eines Polynukleotid-Fragments mit mindestens 18 aufeinanderfolgenden Nukleotiden, dessen Sequenz dem für das Polypeptid BNIPXL-beta der Sequenz von SEQ ID Nr. 1 kodierenden Gens oder dem Transkript BNIPXL-beta der Sequenz von SEQ ID Nr. 2 ganz oder teilweise entspricht, zu kosmetischen Zwecken im Bereich der Pigmentierung zur Vorbeugung vor bzw. Behandlung von Haarergrauung.

11. Verfahren zur Diagnose einer Prädisposition für vorzeitige Haarergrauung bei einer Person, die folgenden Schritte umfassend:
i) Bestimmen der Expressionsebene des Transkripts BNIPXL-beta der Sequenz von SEQ ID Nr. 2 anhand einer Körperprobe der Person,
ii) Vergleichen mit einer Referenzebene, wobei die Referenzebene die Expressionsebene des Transkripts BNIPXL-beta bei einer von vorzeitiger Haarergrauung nicht betroffenen Person ist.

12. Verfahren zum Screening von Molekülen, die die Interaktion zwischen einem Polypeptid BNIPXL-beta der Sequenz von SEQ ID Nr. 1 und einem aus der Translation des Gens DDX31 stammenden Polypeptids modulieren, wodurch das Identifizieren eines Wirkstoffs für eine Verwendung zu kosmetischen oder therapeutischen Zwecken im Bereich der Haarpigmentierung möglich ist.

13. Verfahren nach Anspruch 12,
bei dem die Modulation eine Modifizierung der Bindungskonstante zwischen den beiden Polypeptiden ist.
